# EUROPÄISCHE PATENTSCHRIFT

(11) **EP 0 654 970 B1**
(45) Veröffentlichungstag und Bekanntmachung des Hinweises auf die Patenterteilung: **11.02.1998**
(21) Anmeldenummer: 93917555.0
(22) Anmeldetag: 13.08.1993
(51) Int. Cl.: A61B 1/24, A61B 1/04, A61B 1/05, H04N 7/18

(54) **VIDEOKAMERA ZUM BETRACHTEN VON OBJEKTEN IM MUNDE EINES PATIENTEN**
VIDEO CAMERA FOR OBSERVING OBJECTS IN THE MOUTH OF A PATIENT
CAMERA VIDEO D'OBSERVATION D'OBJETS DANS LA BOUCHE D'UN PATIENT

(30) Priorität: 14.08.1992 DE 4226990
(43) Veröffentlichungstag der Anmeldung: 31.05.1995
(73) Patentinhaber: SIEMENS AKTIENGESELLSCHAFT, 80333 München (DE)
(72) Erfinder: FRANETZKI, Manfred, D-64625 Bensheim (DE)
(86) Internationale Anmeldenummer: DE9300732
(87) Internationale Veröffentlichungsnummer: WO9404068

(56) Entgegenhaltungen:
- DE-A- 4 102 196
- US-A- 4 746 203
- US-A- 4 858 001
- US-A- 4 890 159
- US-A- 4 891 696
- PATENT ABSTRACTS OF JAPAN vol. 10, no. 80 (P-441)(2137) 29. März 1986 & JP,60 217 326 (OLYMPUS KOGAKU KOGYO K.K.) 30. Oktober 1985

## Beschreibung

Die Erfindung bezieht sich auf eine als Handstück ausgebildete Videokamera zum Betrachten von Objekten im Munde eines Patienten.

Bei Verwendung einer Videokamera, wie sie beispielsweise aus US-PS 4 727 416 oder US-5 049 070 bekannt sind, fehlt naturgemäß a priori am Bildschirm die Information über die Objektlage und Blickrichtung. Das Objekt erscheint stets auf der gleichen Stelle mit gleicher Ausrichtung auf dem Schirm.

Aus EP-0 389 453 ist ein für den zahnärztlichen Einsatz vorgeschlagenes Endoskop bekannt, bei dem das Bild über eine Faseroptik aufgenommen und einer Telekamera zugeführt wird. Die Telekamera ist extern des Handinstruments angeordnet und liefert die dort gewonnenen Bildinformationen an einen Monitor weiter. Das in den Patientenmund einführbare Endstück des Handinstruments kann gegenüber der Faseroptik um deren Längsachse gedreht werden. Damit ist es möglich, die Bilder auf dem Monitor aufrecht wiederzugeben, ohne daß hierzu das Verbindungskabel mit der Faseroptik zwischen Handstück und Kamera gedreht zu werden braucht. Das Objekt erscheint auf dem Monitor an der gleichen Stelle mit gleicher Ausrichtung auf dem Bildschirm, d.h. ein Zahn in der linken Hälfte des Unterkiefers erscheint auf dem Monitor gleichermaßen auf der linken Seite, jedoch spiegelverkehrt. Das gleiche gilt für den Oberkiefer. Der Zahnarzt sieht also das Bild auf dem Monitor so, wie er es mit einem kleinen Spiegel, mit dem er im Patientenmund das Objekt betrachtet, sehen würde.

Das gleiche gilt auch für das aus der US-A-4 890 159 bekannte Endoskopie-System, welches eine Vielzahl von unterschiedlich gestalteten Endoskopen umfaßt, die alternativ an eine zentrale Bildverarbeitungseinheit anschließbar sind. Die Elektronik der Bildverarbeitungseinheit ist so ausgebaut, daß am Monitor ein aufrecht oder spiegelbildliches Videobild wiedergegeben werden kann, unabhängig davon, ob man am Eingang der Bildverarbeitungseinheit ein Endoskop mit frontaler oder seitlicher Bilderfassungsoptik oder ein Endoskop mit langem oder kurzem Bildwandler angeschlossen ist.

Aus US-PS 4 858 001 ist es bekannt, das Endstück des Handinstruments gegenüber der übrigen Optik drehbar auszubilden, um so das Objekt auf dem Bildschirm relativ zum Betrachter gedreht darstellen zu können. Mit einer solchen Anordnung ist es zwar möglich, die Seitenposition richtig wiederzugeben, aber nicht die Position distal/proximal bei Wechsel von Unter- zu Oberkiefer oder vestibulär zu lingual wirklichkeitsnah wiederzugeben.

Das gleiche gilt bei Anwendung eines aus der Fachliteratur (MARTINEZ, R.T.: Image Rotation, IN: IBM Technical Disclosure Bulletin, Vol. 27, No. 1B, June 1984, Seiten 510/511) bekannten Rechenprogrammes, in welchem vorgeschlagen wird, Bildraster auf elektronischem Wege mit Hilfe eines Computerprogrammes elektronisch zu drehen.

Aus US-PS 3 976 982 ist eine Einrichtung bekannt, die es ermöglicht, Bilder auf elektronischem Wege zu manipulieren, indem die Bilder, die in Form von zweidimensionalen Arrays repräsentiert werden, einer Elektronik zugeführt werden, in der die Bilder auf elektronischem Wege gedreht oder gespiegelt werden können.

Für einfache Inspektionen mögen solche Betrachtungen akzeptabel sein. Wenn allerdings bei indirekter Behandlung unter Beobachtung des Bildschirmes ein Instrument geführt werden soll, ist es für den Anwender schwierig, Arbeiten im Patientenmund mit der Kamera und mit Blick ausschließlich auf den Monitor durchzuführen. Ein wechselweises Arbeiten mit Blick auf den Monitor und die Kamera würde ein ständiges Umgewöhnen bedeuten.

Der im Ansprüch 1 angegebenen Erfindung liegt die Aufgabe zugrunde, demgegenüber eine Verbesserung zu erzielen.

Gemäß Anspruch 1 sind Objektiv und Bildwandler gemeinsam drehbar oder das Objektiv drehbar auf einem nicht drehbaren Teil des Handstückes, welcher den Bildwandler (z.B. CCD) der Kamera enthält, angeordnet, wozu Mittel zur Erfassung der Drehposition des Objektivs vorhanden sind. Es ist ferner eine Einrichtung vorhanden, welche das Bild auf einem Monitor unabhängig von der Blickrichtung der Kamera aufrecht und seitenrichtig wiedergibt, wobei entweder in der Kamera eine weitere Einrichtung zum Erfassen der Blickrichtung der Kamera eingebaut ist oder eine weitere Einrichtung vorhanden ist, mit der die Blickrichtung der Kamera von Hand eingegeben werden kann. Schließlich ist eine Einrichtung vorgesehen, mit der das Bild auf dem Monitor gedreht oder gespiegelt werden kann. Das Objekt kann so wirklichkeitsnah, also praktisch so, wie der Arzt es bei unbewaffnetem Blick in den Mund sieht, dargestellt werden.

Konkret bedeutet dies, daß z.B. bei einer Bewegung der Bewegung der Kamera nach links sich das Bild auf dem Bildschirm nach links bewegt, gleich ob etwa eine Okklusion im Ober- oder Unterkiefer betrachtet wird. Bei einer Bewegung nach unten läuft das Bild auf dem Schirm nach unten, gleich ob von vestibulär oder lingual auf den Zahn geblickt wird. Bei einer Bewegung nach distal bewegt sich eine Okklusionsfläche auf dem Schirm stets in die gleiche Richtung, z.B. nach oben, gleich ob Ober- oder Unterkiefer betrachtet wird.

Die Erfindung zeigt drei Lösungen auf, wobei bei allen drei Ausführungen eine Kamera mit aufgesetztem, gestrecktem, endoskopähnlichem Objektiv verwendet wird.

### Lösung 1

Als Bildwandler ist ein CCD vorgesehen, der nicht drehbar mit einem ebenfalls nicht drehbaren Kabel verbunden ist. Das Objektiv ist in 4 um 90° gedrehten Positionen rastbar. Die Rastpositon wird durch einen Geber, z.B. einen Schaltkontakt, erkannt und zu einer Steuerelektronik geleitet.

Das Bild wird in zwei Raststellungen unverändert, in den beiden anderen gespiegelt verarbeitet (z.B. durch Umkehr der Ausleserichtung des Bildspeichers).

### Lösung 2

Bei dieser wird die Bildspiegelung nicht elektronisch, wie bei der Lösung 1, sondern durch wahlweises Ein- oder Ausklappen eines Umkehrprismas oder eines Spiegels in den Strahlengang des Objektives auf an sich bekannte Weise erreicht.

Analog können diese Lösungen auch in Verbindung mit einem Faserbündel, dessen Biegerichtung mit einem Sensor erfaßt wird, realisiert werden.

### Lösung 3

Bei dieser Ausführung dreht sich der Bildwandler mit dem Objektiv, d.h. Bildwandler (z.B. CCD) und Objektiv sind fest miteinander gekoppelt. Der Blickwinkel der Kamera zum Lot wird erfaßt und das Bild entsprechend mit elektronischen Mitteln auf dem Bildschirm gedreht. Die Erfassung des Drehwinkels erfolgt durch einen Winkelgeber, wobei die Lotrichtung durch die Schwerkraft eines an dessen Achse drehbar exzentrisch befestigten Gewichtes vorgegeben ist. Der Winkelgeber kann z.B. ein Drehpotentiometer oder ein digitaler Geber (Inkrementalgeber) sein. Die Achse des Winkelgebers liegt parallel zur Längsachse des Objektives. Die Drehung des Bildes erfolgt entsprechend dem Signal des Winkelgebers durch Nachverarbeitung des aus dem CCD in den Bildspeicher eingelesenen Primärbildes per Hard- und/oder Softwaremittel.

Die Winkelmessung und entsprechende Bilddrehung kann kontinuierlich erfolgen oder bevorzugt nur eine Detektion der 4 Quadranten sein mit entsprechenden elektronischen Spiegelungen des Bildes (Vertauschen der Ausleserichtung von Zeilen oder Spalten im CCD oder im Bildspeicher).

Die automatische Detektion der 4 Stellungen, Blickrichtung nach oben, unten, links und rechts, kann im einfacheren Falle ersetzt werden durch eine manuelle Eingabe am Gerät, z.B. mittels eines 4-Positions-Schalters.

Gemäß weiterer Ausgestaltung ist eine Vorab-Bildspiegelung vorgesehen, die nicht eingeschaltet ist, wenn der Behandler vor dem Patienten sitzt und der Bildschirm sich hinter dem Patienten befindet (Arbeit in direkter Sicht) und eingeschaltet ist, wenn der Arzt hinter dem Patienten sitzt und der Bildschirm über dem Patienten sich befindet (Arbeiten im Spiegel).

Diese Spiegelung kann wieder optisch im Objektiv oder elektronisch (Ausleseumkehr der Spalten) erfolgen.

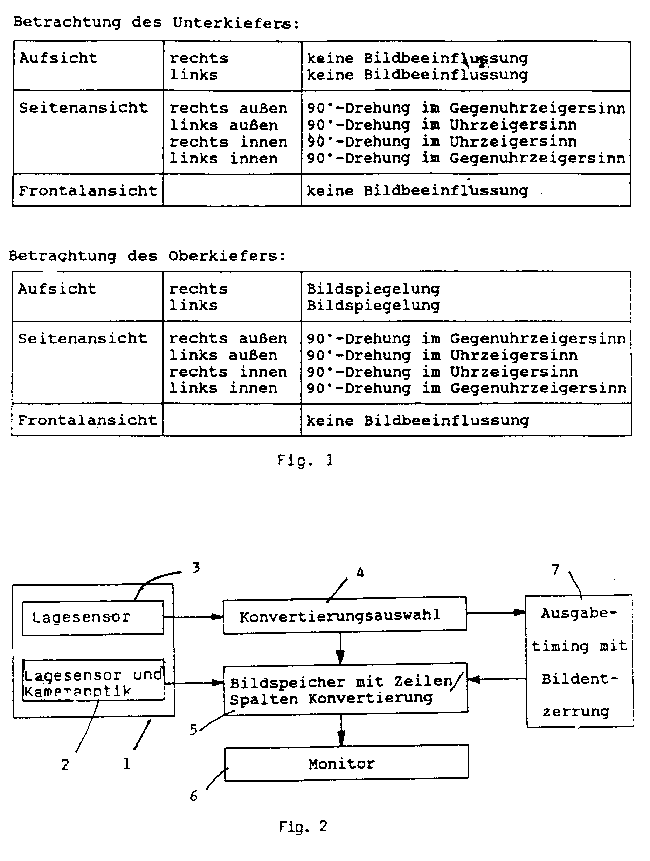

Anhand der Figur 1 wird die Echtzeitbildbehandlung einer lageadaptierten Intraoral-Kamera bei einem aufrecht sitzenden Patienten und einer 8°° Uhr bis 9°° Uhr-Betrachtung einerseits des Oberkiefers und andererseits des Unterkiefers aufgezeigt. Aus der Übersicht geht hervor, daß bei einer Draufsicht auf den Zahn keine Bildbeeinflussung notwendig ist, d.h. ein Zahn bzw. eine Zahnreihe im rechten Kieferbogen wird am Bildschirm so abgebildet, wie ihn die Kamera bz. ein Betrachter in der Aufsicht sieht. Gleiches gilt für die Betrachtung der linken Hälfte des Unterkieferbogens.

Bei Seitenansicht erfolgt je nach Betrachtung (rechts/links - außen/innen) eine Bilddrehung um 90° im Uhrzeigersinn oder im Gegenuhrzeigersinn . Bei Frontalansicht erfolgt, wie leicht einzusehen ist wiederum keine Bildbeeinflussung.

Analoges gilt für die Betrachtung des Oberkiefers.

Anhand des Blockschaltbildes gemäß Figur 2 wird die Funktion und das Zusammenwirken der vorgesehenen Mittel näher erläutert.

Die allgemein mit 1 bezeichnete Kamera enthält einen mit der Pos. 2 bezeichneten Bildsensor mit Elektronik, einer Optik mit Beleuchtung und einem mit Pos. 3 gekennzeichneten Lagesensor, der vier diskrete Kameralagen, vorzugsweise in Stufen von 90°, erkennt.

Der Block "Konvertierungsauswahl" (Pos. 4) erzeugt aus den Signalen des Lagesensors 3 für einen Bildspeicher 5 geeignete Signale. Dadurch wird der Bildspeicher so konfiguriert, daß die jeweilig erforderliche Adaption (Drehung, Spiegelung entsprechend Tabelle in Fig. 1) stattfindet.

Der Bildspeicher 4 hat zwei Aufgaben; er dient zum einen als Zwischenspeicher, zum anderen, um am Monitor 6 ein Standbild zu erzeugen. Eine Zwischenspeicherung ist zur Drehung oder Spiegelung eines "Realtime-Bildes erforderlich. Das manipulierte Bild wird dabei um ein Bild zeitlich versetzt dargestellt.

Ein Standbild entsteht durch permanentes Auslesen, wobei ein gespeichertes Bild im Speicher bleibt, ohne daß ein neues Bild eingelesen wird.

Der mit Pos. 7 bezeichnete Block "Ausgabe-Timing mit Bildentzerrung" ist nur erforderlich bei Drehung um ± 90°. Da das Monitor- bzw. Sensorbild in der Regel ein Seitenverhältnis von 4 : 3 aufweist, ist bei Drehung um ± 90° eine Anpassung an den Monitor erforderlich. Daraus ergeben sich prinzipiell zwei verschiedene Anpassungsformen, nämlich eine Anpassung an die beiden seitlichen Bildränder und an den oberen und unteren Bildrand. Während im einen Fall ein komlettes Bild schwarze Streifen an den Rändern ergibt, gehen bei einem vergrößerten Bild Bildinformation am oberen und unteren Rand verloren, wobei aber die Monitorfläche voll ausgenutzt wird. Da in beiden Fällen eine Maßstabsänderung erfolgt (Vergrößerung, Verkleinerung) muß über den Block 7 das Ausgabetiming entsprechend geändert werden.

## Patentansprüche

1. Als Handstück ausgebildete Videokamera zum Betrachten von Objekten im Munde eines Patienten, bei der Objektiv und Bildwandler gemeinsam drehbar oder das Objektiv drehbar auf einem nicht drehbaren Teil des Handstückes, welcher den Bildwandler (z.B.CCD) der Kamera enthält, angeordnet sind, wobei hierzu Mittel zur Erfassung der Drehposition des Objektivs vorhanden sind, und bei der eine Einrichtung vorhanden ist welche das Bild auf einem Monitor unabhängig von der Blickrichtung der Kamera aufrecht und seitenrichtig wiedergibt, wobei entweder in der Kamera eine weitere Einrichtung zum Erfassen der Blickrichtung der Kamera eingebaut ist oder eine weitere Einrichtung vorhanden ist, mit der die Blickrichtung der Kamera von Hand eingegeben werden kann, und daß eine Einrichtung vorgesehen ist, mit der das Bild auf dem Monitor gedreht oder gespiegelt werden kann.

2. Videokamera nach Anspruch 1, **dadurch gekennzeichnet,** daß elektronische Einrichtungen vorgesehen sind.

3. Videokamera nach Anspruch 1, **dadurch gekennzeichnet,** daß im Strahlengang der Kamera eine opto-mechanische Einrichtung, z.B. klappbare Umkehrprismen oder Spiegel, vorgesehen ist.

4. Videokamera nach Anspruch 1, **dadurch gekennzeichnet,** daß die Einstellung und Erfassung in vier Grundrichtungen nach unten, oben, links oder rechts erfolgt.

5. Videokamera nach Anspruch 1, **dadurch gekennzeichnet,** daß die Drehposition in vier Raststellungen mit einer elektronischen Einrichtung, z.B. einem Mikroschalter oder einer Lichtschranke, erfaßt wird.

6. Videokamera nach Anspruch 1, **dadurch gekennzeichnet,** daß die Drehposition kontinuierlich mit einem Winkelgeber (Potentiometer, digitaler Geber) erfaßt wird.

7. Videokamera nach Anspruch 1, **dadurch gekennzeichnet,** daß der Winkel des Objektivs mit einem Winkelgeber gemessen wird, der jeweils den Winkel zum Lot erfaßt.

8. Videokamera nach Anspruch 7, **dadurch gekennzeichnet,** daß der Winkelgeber aus einem an der Achse eines Potentiometers oder digitalen Winkelgebers exzentrisch befestigten Gewicht besteht.

## Claims

1. Video camera constructed as a handpiece for observing objects in the mouth of a patient, where the objective and the image transducer are together rotatably arranged, or the objective is rotatably arranged on a non-rotatable part of the handpiece which contains the image transducer (for example CCD) of the camera, whereby for this purpose means are present to acquire the rotational position of the objective, and where a device is present which reproduces the image on a monitor, independently of the viewing direction of the camera, so that it is erect and non-reversed, whereby either an additional device is built into the camera in order to acquire the viewing direction of the camera or another device is present with which the viewing direction of the camera can be entered manually, and in that a device is provided with which the image can be rotated or mirrored on the monitor.

2. Video camera according to claim 1, characterized in that electronic devices are provided.

3. Video camera according to claim 1, characterized in that an opto-mechanical device, for example hinged reversing prisms or mirrors, is provided in the beam path of the camera.

4. Video camera according to claim 1, characterized in that the setting and acquisition takes place in four fundamental directions of downward, upward, left or right.

5. Video camera according to claim 1, characterized in that the rotational position in four locked positions is acquired with an electronic device, for example a microswitch or a light barrier.

6. Video camera according to claim 1, characterized in that the rotational position is acquired continuously with an angle transmitter (potentiometer, digital sensor).

7. Video camera according to claim 1, characterized in that the angle of the objective is measured by an angle transmitter which acquires the angle to the perpendicular in each case.

8. Video camera according to claim 7, characterized in that the angle transmitter comprises a weight which is eccentrically secured to the axis of a potentiometer or digital angle transmitter.

## Revendications

1. Caméra vidéo réalisée en pièce à main pour observer des objets dans la bouche d'un patient, dans laquelle l'objectif et le transformateur d'images sont montés tournant conjointement ou dans laquelle l'objectif est monté tournant sur une pièce qui ne tourne pas de la pièce à main qui contient le transformateur d'images (par exemple DCC) de la caméra, des moyens de détection de la position de rotation de l'objectif étant présents à cet effet, et dans laquelle il est présent un dispositif qui reproduit verticalement et du bon côté l'image sur un moniteur indépendamment de la direction de vision de la caméra, un dispositif supplémentaire étant intégré à la caméra pour détecter la direction de vision de la caméra ou un dispositif supplémentaire, par lequel la direction de la caméra peut être entrée manuellement, étant présent et dans laquelle il est prévu un dispositif par lequel l'image peut être tournée ou réfléchie sur le moniteur.

2. Caméra vidéo suivant la revendication 1, caractérisée en ce qu'il est prévu des dispositifs électroniques.

3. Caméra vidéo suivant la revendication 1, caractérisée en ce qu'il est prévu dans la marche des rayons de la caméra un dispositif optomécanique, par exemple des prismes d'inversion ou des miroirs escamotables.

4. Caméra vidéo suivant la revendication 1, caractérisée en ce que le réglage et la détection s'effectuent dans quatre directions de base : vers le bas, vers le haut, vers la gauche ou vers la droite.

5. Caméra vidéo suivant la revendication 1, caractérisée en ce que la position de rotation est détectée en quatre positions d'encliquetage par un dispositif électronique, par exemple par un micro-interrupteur ou par une cellule photoélectrique.

6. Caméra vidéo suivant la revendication 1, caractérisée en ce que la position de rotation est détectée en continu par un indicateur d'angle (potentiomètre, indicateur numérique).

7. caméra vidéo suivant la revendication 1, caractérisée en ce que l'angle de l'objectif est mesuré par un indicateur d'angle qui détecte chaque angle par rapport à la verticale.

8. Caméra vidéo suivant la revendication 7, caractérisée en ce que l'indicateur d'angle est constitué d'un poids fixé de manière excentrée à l'axe d'un potentiomètre ou d'un indicateur d'angle numérique.
